**Europäisches Patentamt**

(19) **European Patent Office**

**Office européen des brevets**

(11) Veröffentlichungsnummer: **0 054 635**
**B1**

(12) **EUROPÄISCHE PATENTSCHRIFT**

(45) Veröffentlichungstag der Patentschrift:
13.02.85

(51) Int. Cl.⁴: **A 61 K 33/14** // (A61K33/14,
31/70, 31/405, 31/415, 31/19)

(21) Anmeldenummer: 81108101.7

(22) Anmeldetag: 09.10.81

(54) **Protektive Lösung für Herz und Niere und Verfahren zu deren Herstellung.**

(30) Priorität: 23.12.80 CH 9510/80

(43) Veröffentlichungstag der Anmeldung:
30.06.82 Patentblatt 82/26

(45) Bekanntmachung des Hinweises auf die Patenterteilung:
13.02.85 Patentblatt 85/7

(84) Benannte Vertragsstaaten:
AT BE CH DE FR GB IT LI NL SE

(56) Entgegenhaltungen:
EP - A - 0 012 272

CHEMICAL ABSTRACTS, Band 81, Nr. 13, 30. September
1974, Zusammenfassung Nr. 75616m, Seite 279
COLUMBUS OHIO (US)
CHEMICAL ABSTRACTS, Band 91, Nr. 17, 22. Oktober
1979, Zusammenfassung Nr. 134134j, Seite 62
COLUMBUS OHIO (US)

*Die Akte enthält technische Angaben, die nach dem
Eingang der Anmeldung eingereicht wurden und die
nicht in dieser Patentschrift enthalten sind.*

(73) Patentinhaber: **Dr. Franz Köhler Chemie GmbH, Neue
Bergstrasse 3 - 7, D-6146 Alsbach-Hähnlein 1 (DE)**

(72) Erfinder: **Bretschneider, Hans-Jürgen, Prof. Dr. med.,
Kolbergerstrasse 10, D-3406 Bovenden (DE)**

(74) Vertreter: **Zutter, Hans Johann Niklaus, EPROVA AG
Forschungsinstitut Im Laternenacker 5,
CH-8200 Schaffhausen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung betrifft eine verbesserte Lösung zur Protektion von Herz und Niere und anderen Organen gegenüber Schädigungen durch einen Durchblutungsstopp bei Operationen und Transplantationen dieser Organe sowie das Verfahren zur Herstellung einer solchen Lösung.

Neue operative Verfahren zur Rekonstruktion schwerer angeborener Herzmißbildungen, Verbesserungen der Herz-Klappen-Prothesen und Fortschritte in der Chirurgie der Herzkranzgefäße haben Möglichkeiten zur vollständigen Wiederherstellung Schwerst-Kranker eröffnet. Während die Herz-Lungen-Maschinen-Technik die Ergebnisse komplizierter Herzoperationen kaum noch behindert, ist die Ischämie-Toleranz — die Toleranz des Organs gegenüber einer vollständigen Unterbrechung seiner Blut- und Sauerstoffversorgung bei Abschaltung aus dem Gesamtkreislauf — bis heute ein bedeutender limitierender Faktor.

Nach vollständiger Unterbrechung der Blut- und Sauerstoffzufuhr wird der Energiehaushalt und die Leistungsfähigkeit des Herzens rasch gestört. Daher wird von manchen Herzchirurgen die Methode der mehr oder weniger kontinuierlichen separaten Versorgung der Herzkranzgefäße mit Blut mittels spezieller Pumpen und Kanülen praktiziert. Diese Koronarperfusionstechnik ist jedoch auch mit schwerwiegenden Nachteilen verbunden: die Hauptäste der Herzkranzgefäße lassen sich nicht immer vollzählig kanülieren, die Koronar-Katheter und das mit Blut versorgte Organ behindern das Operationsfeld und das schlagende Herz macht die Anwendung mikrochirurgischer Methoden mit dem Operationsmikroskop unmöglich.

Daher wurde frühzeitig die Technik des künstlichen Herzstillstandes mit totaler Unterbrechung der Blutzufuhr entwickelt. Das ischämisch stillgelegte Herz überlebt — je nach Vorschädigung und Temperatur — 20—40 Minuten. Die dabei auftretende Erschöpfung der Energiereserven macht häufig eine längere Erholungsdauer mit Entlastung des Herzens durch die Herz-Lungen-Maschine und eine intensive Überwachung der in den ersten postoperativen Tagen stärker gefährdeten Patienten erforderlich. Außerdem steht der Operateur bei diffizilen und komplizierten Eingriffen unter erheblichem Zeitdruck, da ein Überschreiten der sehr begrenzten Ischämie-Toleranzzeit unter allen Umständen vermieden werden muß.

Die limitierte Operationsdauer im rein ischämischen Herzstillstand war der Anlaß für zahlreiche Versuche zur Verbesserung der Myocard-Protektion, z. B. durch Acetylcholin, durch Magnesiumsalze, durch Novocain und durch die von H. J. Bretschneider 1964 beschriebene Natrium-arme, Calciumfreie, Novocain-haltige kardioplegische Lösung (H. J. Bretschneider: Überlebenszeit und Wiederbelebungszeit des Herzens bei Normo- und Hypothermie; Verh. Dtsch. Ges. Kreislaufforschg., 30 (1964), II; H. J. Bretschneider, G. Hübner, D. Knoll, B. Lohr, H. Nordbeck und P. G. Spieckermann: Myocardial resistance and tolerance to ischemia: Physiological and biochemical basis; J. of Cardiovasc. Surg., 16 (1975), 241). Obwohl diese kardioplegische Lösung eine etwa doppelte Ischämie-Toleranzzeit gegenüber der beim rein ischämischen Herzstillstand garantiert, so ist es aufgrund der großen Fortschritte der operativen Technik doch dringlich geworden, daß die vom Herzen gut tolerierte Ischämiedauer noch weiter verlängert wird und auf 100 bis 120 Minuten bei 30° C ausgedehnt werden kann. Dabei muß die notwendige Erholungszeit möglichst kurz bleiben; nur etwa 10 Minuten, um die gesamte Dauer des Einsatzes der Herz-Lungen-Maschine so kurz als möglich zu halten.

In der europäischen Patentanmeldung 12 272 ist eine protektive Lösung für Herz und Niere und andere Organe beschrieben, welche durch ein Puffersystem auf der Basis von Histidin + Histidin · Hydrochlorid und Tryptophan gekennzeichnet ist und welche außerdem Natrium-, Kalium- und Magnesium-Ionen sowie ein Polyol oder einen Zucker enthält. Mit einer solchen protektiven Lösung kann eine Ischämie-Toleranzzeit erreicht werden, die einen Verbesserungsfaktor von 4—8 gegenüber dem unbeeinflußten Herzen aufweist.

Zweck der vorliegenden Erfindung ist, eine weitere energetische und metabolische Verbesserung der Organprotektion zu erreichen.

Es wurde nun gefunden, daß sich der quantitative Leistungsgrad der in der oben genannten Patentanmeldung beschriebenen protektiven Lösung durch einen Zusatz von $\alpha$-Ketoglutarat signifikant steigern läßt.

Auf einen Zusatz von Lithium-Ionen kann verzichtet werden. Die bisherigen hohen Konzentrationen des Puffers Histidin/Histidin · Hydrochlorid können durch $\alpha$-Ketoglutarat teilweise ersetzt werden.

Der Zusatz von $\alpha$-Ketoglutarat zu der organprotektiven Lösung verbessert den aeroben Stoffwechsel während der ca. 10 Minuten langen Perfusion des Organs mit der protektiven Lösung. Diese Verbesserung der Aerobiose geht ohne eine Steigerung des Grundumsatzes einher, sie beruht wahrscheinlich z. T. auf einer Unterdrückung ungünstiger lipolytischer Vorgänge sowie auf einer Hemmung unerwünschter lipolytischer Transaminierungsprozesse. Durch die Einschleusung des $\alpha$-Ketoglutarates in den Citratzyklus wird relativ NADH (Nicotinyl-Adenin-Dehydrogenase) eingespart und damit eine besonders günstige Ausgangssituation für die folgende Ischämiezeit geschaffen.

Die verbesserte erfindungsgemäße protektive Lösung zur Verhinderung von Ischämie-Schäden an Herz und Nieren und weiteren Organen wie Leber, Muskel, Zentralnervensystem bei Operationen oder Transplantationen dieser Organe ist dadurch gekennzeichnet, daß sie pro Liter folgende Zusätze enthält:

| | |
|---|---|
| Kalium- oder Natriumhydrogen-$\alpha$-ketoglutarat | 4±3 Millimol |
| Natriumchlorid | 15±8 Millimol |
| Kaliumchlorid | 10±8 Millimol |
| Magnesiumchlorid | 10±2 Millimol |
| Tryptophan | 2±1 Millimol |
| Histidin | 150±100 Millimol |
| Histidin · Hydrochlorid | 16±11 Millimol |
| Mannitol | 50±50 Millimol und/oder |
| Fruktose | 50±50 Millimol und/oder |
| Ribose | 50±50 Millimol und/oder |
| Inosin | 50±50 Millimol |

wobei die Osmolarität der Lösungen etwa 300 bis 350 mosm beträgt und das pH zwischen 6,8 bis 7,4 liegt.

Das Verfahren zur Herstellung einer Lösung zur Verhinderung von Ischämie-Schäden an Herz und Nieren und anderen Organen bei Operationen und Transplantationen der Organe ist dadurch gekennzeichnet, daß man pro 1 Liter Wasser folgende Komponenten auflöst:

4±3 Millimol Kalium- oder Natrium-hydrogen-$\alpha$-ketoglutarat, 15±8 Millimol Natriumchlorid, 10±8 Millimol Kaliumchlorid und 10±2 Millimol Magnesiumchlorid, 2±1 Millimol Tryptophan, 150±100 Millimol Histidin, 16±11 Millimol Histidin · Hydrochlorid, sowie mindestens ein Polyol oder mindestens ein Zucker zusetzt, wobei die Osmolarität der Lösung auf etwa 300—350 mosm gehalten und das pH der Lösung auf 6,8 bis 7,4 eingestellt wird.

## Beispiel 1

### Protektive Lösung für Herz und Niere und andere transplantationsfähige Organe

Im Liter Aqua dest. rec. werden aufgelöst:

| | | | |
|---|---|---|---|
| Kaliumhydrogen-$\alpha$-ketoglutarat | 4 mmol | = | 8 mosm |
| Natriumchlorid | 15 mmol | = | 30 mosm |
| Kaliumchlorid | 10 mmol | = | 20 mosm |
| Magnesiumchlorid | 10 mmol | = | 30 mosm |
| Histidin | 150 mmol | = | 150 mosm |
| Histidin · Hydrochlorid | 16 mmol | = | 32 mosm |
| Tryptophan | 2 mmol | = | 2 mosm |
| Mannitol | 50 mmol | = | 50 mosm |
| Osmolarität | | | 322 mosm |

## Beispiel 2

### Protektive Lösung für Herz und Niere

Im Liter Aqua dest. rec. werden aufgelöst:

| | | | |
|---|---|---|---|
| Natriumhydrogen-$\alpha$-ketoglutarat | 1 mmol | = | 2 mosm |
| Natriumchlorid | 15 mmol | = | 30 mosm |
| Magnesiumchlorid | 10 mmol | = | 30 mosm |
| Tryptophan | 2 mmol | = | 2 mosm |
| Histidin | 150 mmol | = | 150 mosm |
| Histidin · Hydrochlorid | 16 mmol | = | 16 mosm |
| Fruktose | 50 mmol | = | 50 mosm |
| Mannitol | 50 mmol | = | 50 mosm |
| Osmolarität | | | 330 mosm |

### Beispiel 3

### Protektive Lösung für Herz und Niere

Im Liter Aqua dest. rec. werden aufgelöst:

| | | | |
|---|---|---|---|
| Kaliumhydrogen-$\alpha$-ketoglutarat | 6 mmol | = | 12 mosm |
| Natriumchlorid | 15 mmol | = | 30 mosm |
| Kaliumchlorid | 10 mmol | = | 20 mosm |
| Magnesiumchlorid | 12 mmol | = | 36 mosm |
| Tryptophan | 2 mmol | = | 2 mosm |
| Histidin | 50 mmol | = | 50 mosm |
| Histidin · Hydrochlorid | 5 mmol | = | 5 mosm |
| Ribose | 75 mmol | = | 75 mosm |
| Inosin | 75 mmol | = | 75 mosm |
| Osmolarität | | | 305 mosm |

Der Fortschritt der erfindungsgemäßen protektiven Lösung gegenüber dem Stand der Technik wird durch die nachfolgende Gegenüberstellung mit vorbekannten kardioplegischen Lösungen nachgewiesen.

### Kardioplegische Vergleichs-Lösungen

1./2. Die bereits 1964 beschriebene kardioplegische Lösung 1 (H. J. Bretschneider, Überlebenszeit und Wiederbelebungszeit des Herzens bei Norma- und Hypothermie; Verh. Dtsch. Ges. Kreislaufforschung. 30. (1964), 11) und die 1967 geringfügig modifizierte Lösung 2, welche den vorletzten Stand der Technik charakterisieren, hatten folgende Zusammensetzung:
Im Liter Aqua dest. rec. waren enthalten:

| | Lösung Nr. 1 | Lösung Nr. 2 |
|---|---|---|
| Natriumchlorid | 5 mmol | 12 mmol |
| Kaliumchlorid | 5 mmol | 7 mmol |
| Magnesiumchlorid | — | 1 mmol |
| Glukose | 5,5 mmol | 11 mmol |
| Procain | 8,5 mmol | 8,5 mmol |
| Mannit | 267,5 mmol | 241 mmol |
| Osmolarität | 310 mosm | 310 mosm |
| pH | 7,4 | 7,4 |

3. Die in der europäischen Patentanmeldung 12 272 beschriebene Vergleichslösung, welche den letzten Stand der Technik charakterisiert, hatte folgende Zusammensetzung:
Im Liter Aqua dest. rec. waren aufgelöst:

| | | | |
|---|---|---|---|
| Natriumchlorid | 15 mmol | = | 30 mosm |
| Kaliumchlorid | 10 mmol | = | 20 mosm |
| Lithiumchlorid | 1 mmol | = | 2 mosm |
| Magnesiumchlorid | 1 mmol | = | 3 mosm |
| Histidin | 160 mmol | = | 160 mosm |
| Histidin · Hydrochlorid | 16 mmol | = | 32 mosm |
| Tryptophan | 2 mmol | = | 2 mosm |
| Mannitol | | | 50 mosm |
| | | | 299 mosm |

pH                                                  7,1

Mit den Lösungen Nr. 1., 2. und 3. und mit der erfindungsgemäßen Lösung gemäß vorliegendem Beispiel 2 wurden an Hundeherzen die in der folgenden Tabelle aufgeführten Daten ermittelt:

| Kardioplegische Lösung | Ischämiezeiten in Minuten | | | |
|---|---|---|---|---|
| | bei 35°C | bei 25°C | bei 15°C | bei 5°C |
| Nr. 1 | 70 | 110 | 256 | 415 |
| Nr. 2 | 62 | 142 | 282 | 435 |
| Nr. 3 »HTP« | 100 | 210 | 420 | 800 |
| »HTK« erfindungsgemäße Lösung, Beispiel 2 | 120 | 260 | 520 | 1000 |

Präischämische Äquilibrierung des Herzens mit der Histidin-Tryptophan-Polyol-Lösung Nr. 3 (Lösung »HTP«) vermag im Hunde-Experiment die Ischämietoleranz des Organs gegenüber reinem ischämischen Herzstillstand bei gleicher Temperatur um etwa den Faktor 8 zu verlängern (Bretschneider, H. J.: Thorac. Cardiovasc. Surgeon 28, 295 (1980)). Der Zusatz von 1 mmol/Liter Kalium-$\alpha$-ketoglutarat (Lösung »HTK«) erbringt nach biochemischen Analysen in 8 Hundeherzen während Ischämie in Normo- und Hypothermie eine energetische und metabolische Verbesserung der Protektion wie sie in der vorstehenden Tabelle zum Ausdruck kommt.

Es wird darin nachgewiesen, daß mit der durch Zusatz von $\alpha$-Ketoglutarat verbesserten kardioplegischen Lösung die Ischämie-Toleranzzeiten bei 35°C um ca. 20%, bei 25°C um ca. 20%, bei 15°C um ca. 25% und bei 5°C um ca. 25% verlängert werden können. Die sehr gute funktionelle Wiederbelebbarkeit der protektiv behandelten Herzen mit der erfindungsgemäß beschriebenen Lösung wird durch die signifikante Verbesserung der biochemischen Ergebnisse bestätigt. Es wird quantitativ gemessen an den nutzbaren Ischämiezeiten, d. h. der Zeitspanne, während der der normale ATP-Gehalt des Myokards, gemessen zum Beginn der Ischämie, von etwa 6,5 µMol/g auf den für die Wiederbelebung kritischen und limitierenden Wert von 4,0 µMol/g Herz-Feuchtgewicht abgefallen ist.

In je 5 Wiederbelebungs-Experimenten — 300 min Ischämie bei im Mittel 23°C und anschließender Reperfusion und Wiederbelebung mit physiologischer saliner Tyrode-Lösung — zeigte ein Vergleich der protektiven Lösung Nr. 3 mit derjenigen von Beispiel 2 einen signifikant besseren Energiestatus und damit eine bessere Erholung bei den mit »HTK« protektionierten Herzen (p$\leq$0,05; U-Test nach Wilcoxon, Mann und Whitney):

| Metabolite im Myokard nach Wiederbelebung ($\mu$Mol/g; $x + S_x$) | »HTP«-Lösung Histidin-tryptophan-Polyol gem. Lösung Nr. 3 | »HTK«-Lösung Histidin-tryptophan-$\alpha$-ketoglutarat Lösg. gem. Beisp. 2 |
|---|---|---|
| ATP-Gehalt | 4,1 $\mp$ 0,6 | 4,8 $\mp$ 0,4 |
| Summe der Adeninnukleotide | 5,1 $\mp$ 0,6 | 5,9 $\mp$ 0,5 |

Die mit $\alpha$-Ketoglutarat zusätzlich protektionierten Herzen waren darüber hinaus während der Reperfusion deutlich rhythmusstabiler und weniger ödemanfällig.

Nach diesen Befunden wirkt der Zusatz von 1 mmol/Liter Kaliumhydrogen-$\alpha$-ketoglutarat in der kardioplegischen Lösung im Sinne einer deutlichen Verbesserung der postischämischen metabolischenergetischen Erholung und einer spezifischen Strukturprotektion des ischämischen Organs.

Für eine allgemeinere Gültigkeit dieser Aussage sprechen 5 Hunde-Experimente zur funktionellen Reversibilität einer renalen Ischämie von 100—120 min bei im Mittel 34°C. Die Hunde wurden jeweils einseitig nephrektomiert, die verbleibende Niere mit der Lösung »HTK« äquilibriert und nach der genannten Ischämiebelastung durch Wieder-Freigabe der Blutperfusion »wiederbelebt«. Die Tiere zeigten bereits am 5. postoperativen Tag annähernd normale Kreatinserum-Werte unter 2 mg%; ähnliche Erfolge waren ohne den Zusatz von $\alpha$-Ketoglutarat nicht erreichbar.

Im europäischen Recherchenbericht wird die Arbeit von K. C. Calman, Cryobiology 1974, 11 (1), 7—12 (Chemical Abstracts 81 (1974) 75 616 m) zitiert. Darin wird gezeigt, daß durch Zusatz von hohen Dosen (10 mmol/Liter) einer Kombination von $\alpha$-Ketoglutarat und Oxalessigsäure die Überlebensrate von ischämischen Rattenherzen verbessert wurde. Calman gibt keine Auskunft darüber, wie sich der Zusatz von $\alpha$-Ketoglutarat allein, ohne gleichzeitige Mitwirkung von Oxalsäure, auf die Stabilisierung des ATP-Spiegels auswirkt. Eine Gegenüberstellung der Lösungen von Calman und von Bretschneider gemäß vorliegender Erfindung zeigt die nachfolgende Tabelle.

Die Lösung nach Bretschneider ist ein bewährtes Handelsprodukt.

| | Nach Calman $k_1$ | $k_2$ | Nach Bretschneider |
|---|---|---|---|
| Natrium | 140,0 mmol/l | 70,0 mmol/l | 15,0 mmol/l |
| Kalium | 13,0 mmol/l | 13,0 mmol/l | 9,0 mmol/l |
| Calcium | 0,8 mmol/l | 0,8 mmol/l | — |
| Magnesium | 0,8 mmol/l | 30,0 mmol/l | 9,0 mmol/l |
| Chlorid | 145,0 mmol/l | 70,0 mmol/l | 33,0 mmol/l |
| Phosphat | 10,0 mmol/l | 10,0 mmol/l | — |
| Tris | 10,0 mmol/l | 10,0 mmol/l | — |
| $\alpha$-Ketoglutarsäure | | | 1,0 mmol/l |
| Oxalessigsäure | 10,0 mmol/l | 10,0 mmol/l | — |
| Histidin | — | — | 170,0 mmol/l |
| Histidin · HCl | — | — | 16,0 mmol/l |
| Tryptophan | — | — | 2,0 mmol/l |
| Glukose | 1,0 g/l | 1,0 g/l | — |
| Mannit | — | — | 20,0 mmol/l |
| Dextran 40 | — | 50,0 g/l | — |

Nach Calman werden die Rattenherzen nach Entnahme zunächst mit einer Versuchslösung perfundiert und anschließend in dieser Lösung bei 4 bis 5°C während 12 bis 18 Stunden gelagert.

Während nach dem Verfahren von Calman unter Verwendung von $\alpha$-Ketoglutarat und Oxalsäure bei der Herzkonservierung bei Temperaturen von 4°C nur etwa 50% der Tierherzen überleben, beträgt die Sterblichkeit bei der Anwendung der kardioplegischen Lösung nach Bretschneider in vivo humanem nur 4,4%.

Dabei muß noch berücksichtigt werden, daß die chirurgischen Eingriffe in der Regel zwischen 21 und 27°C Bluttemperatur durchgeführt werden, was eine zusätzliche Belastung der Herzen darstellt. Es ist bekannt, daß allein durch die Hypothermie auf 4 bis 5°C die Herzprotektion um den Faktor 8 verbessert wird.

Allein dieser Vergleich zeigt, daß die erfindungsgemäß beanspruchte kardioplegische Lösung HTK nach Bretschneider einen enormen Fortschritt in Bezug auf die Herzprotektion bei großen offenen Eingriffen erbringt.

Im europäischen Recherchenbericht wird außerdem auf die Publikation von Kh. D. Bairamkulov, Chemical Abstracts 91 (1979) 134134j verwiesen, wonach die Kombination Natriumsuccinat und $\alpha$-Ketoglutarsäure die kollaterale Koronarzirkulation beim experimentell ischämierten Hundeherzen verstärkt.

Auch hier handelt es sich um einen Kombinationseffekt, wobei der Wirkungsanteil der $\alpha$-Ketoglutarsäure nicht erkennbar ist. Außerdem sind die Versuche am schlagenden, arbeitenden Herzen durchgeführt und nicht am stillgelegten Myokard. Insofern sind tatsächlich irgendwelche Vergleiche nicht erlaubt.

**Patentansprüche**

1. Protektive Lösung zur Verhinderung von Ischämie-Schäden an Herz und Nieren, sowie anderen Organen bei Operationen und Transplantationen der Organe, dadurch gekennzeichnet, daß sie pro Liter folgende Zusätze enthält:

| | |
|---|---|
| Kalium- oder Natrium-hydrogen-$\alpha$-ketoglutarat | 4 ± 3 Millimol |
| Natriumchlorid | 15 ± 8 Millimol |
| Kaliumchlorid | 10 ± 8 Millimol |
| Magnesiumchlorid | 10 ± 2 Millimol |
| Tryptophan | 2 ± 1 Millimol |
| Histidin | 150 ± 100 Millimol |
| Histidin · Hydrochlorid | 16 ± 11 Millimol |
| Mannitol | 50 ± 50 Millimol |
| Fruktose | 50 ± 50 Millimol |
| Ribose | 50 ± 50 Millimol |
| Inosin | 50 ± 50 Millimol |

wobei die Osmolarität der Lösung etwa 300 bis 350 mosm beträgt und das pH der Lösung zwischen 6,8 und 7,4 liegt.

2. Verfahren zur Herstellung einer Lösung zur Verhinderung von Ischämie-Schäden an Herz und Nieren und anderen Organen bei Operationen und Transplantationen der Organe, dadurch gekennzeichnet, daß man pro 1 Liter Wasser folgende Komponenten auflöst: 4 ± 3 Millimol Kalium- oder Natrium-hydrogen-$\alpha$-ketoglutarat, 15 ± 8 Millimol Natriumchlorid, 10 ± 8 Millimol Kaliumchlorid und 10 ± 2 Millimol Magnesiumchlorid, 2 ± 1 Millimol Tryptophan, 150 ± 100 Millimol Histidin, 16 ± 11 Millimol Histidin · Hydrochlorid, sowie mindestens ein Polyol oder einen Zucker zusetzt, wobei die Osmolarität der Lösung auf etwa 300—350 mosm gehalten und das pH der Lösung auf 6,8 bis 7,4 eingestellt wird.

**Claims**

1. Protective solution for preventing ischaemia damage to the heart and kidneys and also other organs in operations and in organ transplant work, characterised in that it contains the following additives per litre:

| | |
|---|---|
| potassium or sodium hydrogen $\alpha$-ketoglutarate | 4 ± 3 millimols |
| sodium chloride | 15 ± 8 millimols |
| potassium chloride | 10 ± 8 millimols |
| magnesium chloride | 10 ± 2 millimols |
| trytophane | 2 ± 1 millimol |
| histidine | 150 ± 100 millimols |
| histidine hydrochloride | 16 ± 11 millimols |
| mannitol | 50 ± 50 millimols |
| fructose | 50 ± 50 millimols |
| ribose | 50 ± 50 millimols |
| inosine | 50 ± 50 millimols |

the osmolarity of the solution amounting to approximately 300 to 350 mosm, and the pH of the solution being between 6.8 and 7.4.

2. Process for the production of a solution for preventing ischaemia damage to heart and kidneys and other organs in operations and in organ transplant work, characterised in that the following constituents are put into solution per 1 litre of water: 4 ± 3 millimols potassium or sodium hydrogen $\alpha$-ketoglutarate, 15 ± 8 millimols sodium chloride, 10 ± 8 millimols potassium chloride and 10 ± 2 millimols magnesium chloride, 2 ± 1 millimol tryptophane, 150 ± 100 millimols histidine, 16 ± 11 millimols histidine hydrochloride, also at least one polyol or a sugar is added, and the osmolarity of the solution is kept to approximately 300—350 mosm, and the pH of the solution is adjusted to 6.8 to 7.4.

**Revendications**

1. Solution protectrice pour inhiber les dommages causés par l'ischémie sur le coeur et les reins ainsi que d'autres organes dans les opérations et transplantations des organes, caractérisée en ce qu'elle contient par litre les additifs suivants:

| | |
|---|---|
| $\alpha$-cétoglutatate acide de potassium ou de sodium | $4 \pm \ \ 3$ millimol |
| Chlorure de sodium | $15 \pm \ \ 8$ millimol |
| Chlorure de potassium | $10 \pm \ \ 8$ millimol |
| Chlorure de magnésium | $10 \pm \ \ 2$ millimol |
| Tryptophane | $2 \pm \ \ 1$ millimol |
| Histidine | $150 \pm 100$ millimol |
| Chlorhydrate d'histidine | $16 \pm \ 11$ millimol |
| Mannitol | $50 \pm \ 50$ millimol |
| Fructose | $50 \pm \ 50$ millimol |
| Ribose | $50 \pm \ 50$ millimol |
| Inosine | $50 \pm \ 50$ millimol |

où l'osmolarité de la solution s'élève à environ 300 à 350 mosm et le pH de la solution se situe entre 6,8 et 7,4.

2. Procédé de préparation d'une solution pour inhiber les dommages causés par l'ischémie sur le coeur et les reins et d'autres organes dans les opérations et transplantations des organes, caractérisé en ce qu'on dissout pour 1 litre d'eau les composants suivants: $4 \pm 3$ millimol d'$\alpha$-cétoglutarate acide de potassium ou de sodium, $15 \pm 8$ millimol de chlorure de sodium, $10 \pm 8$ millimol de chlorure de potassium et $10 \pm 2$ millimol de chlorure de magnésium, $2 \pm 1$ millimol de tryptophane, $150 \pm 100$ millimol d'histidine, $16 \pm 11$ millimol de chlorhydrate d'histidine, ainsi qu'au moins un polyol ou un sucre, l'osmolarité de la solution étant maintenue à environ $300-350$ mosm et le pH la solution à 6,8 à 7,4.